# EUROPEAN PATENT SPECIFICATION

(11) **EP 3 720 521 B1**
(45) Date of publication and mention of the grant of the patent: **10.11.2021**
(21) Application number: 18839919.0
(22) Date of filing: 05.12.2018
(51) Int. Cl.: A61M 5/14, A47F 5/00, A45F 3/04, A45F 3/14, A45C 13/02, A45C 11/00, A61J 1/10, A61J 7/00, A61J 15/00

(54) **PORTABLE PUMP AND CONTAINER CARRIER**
TRAGBARE PUMPE UND BEHÄLTERTRÄGER
POMPE ET SUPPORT DE RÉCIPIENT PORTABLE

(30) Priority: 05.12.2017 NL 2020022
(43) Date of publication of application: 14.10.2020
(73) Proprietor: N.V. Nutricia, 2712 HM Zoetermeer (NL)
(72) Inventor: VAN KINDEREN, Sencha, 3584 CT Utrecht (NL)
(74) Representative: Nederlandsch Octrooibureau
(86) International application number: PCT/NL2018/050813
(87) International publication number: WO 2019/112429

(56) References cited:
- WO-A1-2016/160676
- CA-A1- 2 949 288
- GB-A- 2 395 423
- US-A- 5 236 004
- US-A- 5 630 537
- US-A1- 2013 119 850

## Description

### Field of the invention

The present invention relates to a portable pump and container carrier for transport and administration of enteral food or other liquids.

### Background art

Patients with certain medical problems may require having enteral food or liquids administered in predetermined doses throughout the day. Containers holding the enteral food or liquid come in many different structures and shapes, having different sized and shaped necks and screw fittings. Regular stands allow for a container, such as a bottle or pouch, independent from its shape, to be hung such that the enteral food or liquid can be fed through a feeding set under gravity or by pump. The feeding sets are available in at least the following, different configurations:
- with drip chamber - for use with gravity
- with drip chamber - for use with a pump
- without drip chamber - for use with a pump

In this context, feeding set is understood to generally denote an assembly of tubing with the requisite connectors for connecting to the container on the one side and to the patient feeding tube at the other side, with or without drip chamber. Furthermore, it must be noted that drip chambers may also come in a range of sizes.

In most cases, patients with no or limited mobility will use a feeding set with a drip chamber. Herein, a drip chamber will be situated between the container and the tubing, acting as a micro-bacterial barrier, preventing bacteria traveling up into the container where they can multiply and become harmful to very weak patients. An additional advantage of using a drip chamber is that the drip chamber offers visual feedback of enteral food or liquid being fed through the tubing.

The regular stands referred to above are generally in the form of infusion poles or may be integrated into a bed frame. This has as a drawback that they are very limiting to the freedom of movement for patients who are not bedridden. These patients may be better served with a portable carrier, which can be used as both a stationary frame as well as a mobile frame, carried in a backpack. One stand for enteral feeding sets that can be carried in a backpack is shown in WO2016160676. Another support device for use with a fluid delivery system is shown in US 5 236 004.

The design of a carrier for mobile use needs to be different from regular stands, which due to their size and shape are generally not suitable for fitting in a backpack. Furthermore, whilst being carried in a backpack the container is in a lower position with respect to the patient, thus a feed pump is required to ensure that sufficient enteral food or liquid is fed through the tubing at a controlled and accurate rate. It is also to be noted that although a stand, container and pump may be located within a backpack, there may be a danger of leakage, especially if the backpack is knocked or shaken. Bags and backpacks that can be used for carrying consumables and other items are known from GB 2 395 423, US 5 630 537 and US 2013/119850.

Mobile patients generally use a feeding set without drip chamber inside their backpack. If a drip chamber were present, air could become trapped in the feeding set as a consequence of shaking liquid inside the drip chamber caused movement, which could set off pump alarms. These feeding sets are generally short, for convenient use with a backpack without excess tubing suffering from kinking or forming knots. During the night, however, these patients will often want to switch to a set with drip chamber, comprising longer tubing, which provides a more comfortable length for unrestricted sleeping. It should be noted that another group of patients, such as elderly or disabled patients who are mobile in a wheelchair or with a walker, might not experience alarms from trapped air and choose to use feeding sets with both a drip chamber and a feed pump.

Although a range of portable stands is available to these mobile patients, these known portable stands have as a disadvantage that only certain containers, which may be bottles or pouches, fit into the stand. Furthermore, known portable stands can mostly only be used without a drip chamber and are insufficiently stable when using higher volume containers. Mobile patients will thus often require to have multiple stands and may even be required to acquire a new mobile stand if their prescribed enteral food or the size of the container the food is provided in changes.

The aim of the present invention is to provide an improved portable pump and container carrier that can be used with different sizes and/or shaped containers whilst allowing the use of a range of feeding sets, with or without drip chambers while ensuring stability of the assembly.

### Summary of the invention

According to the invention, there is provided a portable carrier for transporting and administering enteral food or other liquids as defined in claim 1. The carrier comprises a shell, having a bottom tray, a back plate, two side plates and a feed pump holding element. Support elements are provided for holding a container in place within the shell, the support elements comprising a lower support element for location beneath the container, a rear support element for engaging a rear surface of the container and a container retaining element for retaining the container in position within the shell, wherein the support elements are height adjustable with respect to the bottom tray, thereby allowing a container to be supported at different positions above the bottom tray. The shell may also have an open upper side, allowing a container to extend out of the shell, above a top edge of the back plate and/or the side plates.

The height adjustable lower support element allows for a height between the bottom tray and support element to be adjusted such that sufficient space can be made available for various sized and shaped container necks and feeding sets with or without drip chambers. The height adjustable rear support element offers further support and stability to larger containers and/or higher positioned containers, while the adjustability of the container retaining element ensures that this too adapts together with the other support elements or to the size of a longer container. The height adjustable support elements thus facilitate the use of various types of feeding sets, with and without drip chamber. The adjustability of the support elements allows the carrier to be adjusted to be as compact as possible for the container and feeding set it is carrying, ensuring the carrier can comfortably be carried around in a bag. The bag can be a pouch, net, backpack, frontpack, beltpack, holdall, etc.

The container retaining element further ensures the container is secured in place within the carrier, and able to withstand loads occurring from being mobile. The container retaining element may be a strap, band or web. However, other types of retaining elements such as click connections or hook and eye type fasteners can also be used..

The carrier can be used as a mobile stand inside a dedicated bag, specifically designed to fit with the carrier, or used in a bag of personal choice. The shell of the carrier hereby serves as a protective shell for the container and also for the pump and feeding set. The bottom tray may be provided with openings should this be required e.g. for passage of cables or tubes. In a preferred embodiment the bottom tray is embodied as a spillage tray, without openings. Thus the spillage tray is an integral part of the shell, shaped as an indented region, e.g. dish shaped, or with upstanding edges. Through the presence of the spillage tray, the shell may retain spilled enteral food or liquid, helping ensure that any bag used for carrying will remain clean. Furthermore, the bottom tray of the shell is designed to provide a stable base for the carrier inside a bag as well as on any flat surface. As a result, the carrier is also suitable for use as a stationary frame on for example a table or a nightstand.

According to an embodiment, the lower support element comprises a container rest shelf, extending between the side plates, which is repositionable, such that a distance between the shelf and the bottom tray is variable. The shelf may also extend to and/or be affixed to the back plate. The shelf provides a stable support to any container whereby loads resulting from the weight of the container and any additional loads resulting from being mobile are distributed from the container's contact surface to the shelf's attachment points or area on the shell. Furthermore, the shelf acts as a separator within the shell between the container and the bottom tray. The shelf has an opening for bottles having a neck and shoulders, with the shoulders resting on the shelf, such that the neck with the connection to the feeding set can be located in the space above the bottom tray.

The height of the shelf can be adjusted such that sufficient space is available between the shelf and the bottom tray for the tubing of the feeding set not to form a kink. To this end, the side plates each comprise two or more ribs or slots, provided in pairs at two or more heights from the bottom tray, for positioning of the shelf. These heights are set to a space sufficient for most bottles attached to a feeding set without a drip chamber and to a set with a drip chamber. Further pairs of ribs or slots may be provided at intermittent heights, to account for smaller drip chambers or shorter bottle necks.

A further advantage of having the shelf attached to the side plates is that the shelf thereby provides additional stiffness to the carrier shell. Additionally, the shelf may comprise one or more locking elements at a back edge that can engage with the back plate of the shell. The back plate of the shell may comprise e.g. one or more locking element receiving slots located at two or more heights from the bottom tray for receiving the one or more locking elements. These locking element receiving slots may be positioned such that the locking element is locked inside a slot when the shelf is positioned on the ribs or in the slots. Locking the shelf within the shell increases its rigidity and ensures the carrier configuration remains as desired throughout mobile use.

The shelf may be slidably positionable over each set of ribs or into each set of slots by a movement in the direction of the ribs or slots, whereby the ribs or slots extend from the front edge of the side plates towards the back plate. The ribs or slots may be sloped downward towards the back plate to ensure the container will naturally rest against the back support. An angle of at least 5 degrees with respect to the bottom tray has proven to provide the desired effect. To increase ease of use, the shelf may be symmetric about its own plane such that the shelf can be used upside down without issues. Thus a user positioning the shelf within the shell need not pay attention to the orientation of the shelf in this respect and misplacement is avoided. This increases user convenience and reduces user errors. To further allow easy positioning of such a shelf, the shelf may be slightly triangular/trapezoidal in side view and the ribs may also be slightly triangular/trapezoidal to receive it. For an engagement that grips without jamming, an angle between the upper and lower surfaces of the shelf of between 5 degrees and 10 degrees may be chosen.

According to the invention, the rear support element comprises an adjustable container stabilizer, extendable vertically with respect to the back plate to a position above the top edge of the back plate. This enables the carrier to provide sufficient stabilizing support to containers which are sized or placed such that the centre of gravity of the container extends above the shell of the carrier. The adjustable container stabilizer makes the carrier suitable for use with containers of larger size otherwise not properly catered for by portable carrier frames, such as containers larger than 500 ml. Furthermore, this stabilizer allows for the carrier height to be increased only when required. This may be done independently from adjusting the height of the lower support element. Thus when small containers are being used, the height of the carrier can be kept at a minimum, providing ease of transport in a bag.

The stabilizer may be located within or against the back plate such that a longitudinal centreline of the stabilizer is parallel to the back plate, whereby the stabilizer is slidably movable along its centreline, and wherein the back plate further comprises container stabilizer locking elements, located at two or more heights from the bottom tray. The advantage of this configuration is that the stabilizer may be hidden fully within the back plate, thus allowing the carrier size to be limited to the size of the shell. By storing the stabilizer within the back plate, the stabilizer could add further stiffness to the shell. Furthermore, in this configuration the stabilizer can be a single extendable element, thus limiting the total number of components of the carrier and minimizing the overall weight of the carrier. The adjustable container stabilizer may be a plate with lockable elements. Advantageously, a plate-like stabilizer located within the back plate is prevented from rotating around a longitudinal axis, adding further stability to the carrier and keeping the container in the set position.

Alternatively the adjustable container stabilizer may be a wireframe, formed out of a single continuous wire, of which the wire ends are lockable in the stabilizer locking elements. A wireframe could also be used for hanging products onto it, for example when bags are used as containers. An advantage of having a wireframe, formed out of a single wire is that such a wire has no sharp corners or sharp points, making the use of such a wireframe safe for the user and non-damaging to the bag in which the carrier may be transported.

Additionally, the container stabilizer may comprise a hanging hook, to facilitate hanging the carrier from a hook or infusion pole stand. It will be understood that in this case, the container stabilizer locking elements must prevent it from being further withdrawn from the back plate than desired.

To further add to the stability of the carrier, the container stabilizer locking elements may be spaced in pairs, symmetrically around the centreline and located at two or more heights from the bottom tray. The stabilizer locking elements may be simple slots or holes into which the lockable elements of the stabilizer may snap into place. This allows the stabilizer to be configured as an easy to use mechanical system, whereby the stabilizer can manually be pushed out of the locked positions for repositioning. These slots allow for easy, cheap and robust adjustability, wherein the stabilizer simply snaps into the locked positions. This is particularly convenient in the case that the stabilizer is a wire and the lockable elements are the wire ends. The resilience of the wire can be used to keep the lockable elements in the slots.

The back plate may be constructed with a top rib, which partially surrounds the stabilizer at the rear. The top rib may be positioned such that the stabilizer passes between a main surface of the back plate and the top rib. Such a top rib construction restricts the stabilizer to move primarily in a vertical direction, while simultaneously giving stiffness to the top edge of the shell to prevent bending e.g. when loaded with a container or subject to external forces.

According to a further embodiment, the back plate of the shell comprises a first set of strap slots and a second set of strap slots, the sets spaced in pairs around and along the longitudinal centreline of the shell, and wherein a distance between the centreline and the first set of strap slots is larger than a distance between the centreline and the second set of strap slots. The container retaining element may comprises at least one strap, connectable through a pair of strap slots, for encircling the container. Thus the strap slots may be set at different widths, allowing the container retaining element to more effectively hold various sized containers. The variable location of the container retaining element allows the carrier to accommodate various containers, including 125 ml bottles, 2 L pouches and baby-bottles.

Additionally, the container retaining element may further comprise at least one vertical strap, which is attached to the adjustable container stabilizer, and wherein the strap is adaptable to the size and shape of the container. An advantage of this configuration is that the strap moves automatically while adjusting the stabilizer.

In another embodiment the carrier further comprises an adjustable footrest, the foot rest being adjustable between a first position, wherein the footrest is within a perimeter of the shell, and a second position, wherein the footrest extends out from the shell, to a position where it can engage a surface on which the shell is placed, to stabilise the carrier. The adjustable footrest can be extended for use when the carrier is used as a stationary frame on for example a bed stand or table, outside a bag. As will be further described below, the shell is shaped to have a height greater than its base in order to adequately receive all of the elements that it is intended to support Advantageously, the extended footrest provides the carrier with a larger footprint, which adds to the stability of the shell. This is especially desirable when the carrieris used in combination with large containers, which cause the carrier to be top heavy and when the stabilizer is extended to a maximum.

The footrest may comprise a wire frame having dimensions corresponding to a horizontal cross-section of the shell above the bottom tray. Such wire frame is easy to store within the shell when not in use and due to its minimal dimensions, adds minimum weight to the carrier. Having a wire frame contributes to the carrier being lightweight, making it more comfortable for carrying around. For a carrier bottom tray with a square or rectangular shape, the wire frame may have a U-shape with diverging legs and a cross-piece. The legs may be stored within the shell and exit the back of the shell, with the cross-piece being stored on the outside of the shell, lying adjacent to or in a recess within the back surface of the back plate.

The shell may further comprise guides within which the wire frame can slide and footrest locking points into which ends of the wire frame can locate. The guides define a path of the wire frame between the stored position and the extended, stabilising position. Extending the footrest is a simple manual operation whereby a user pulls on the accessible part of the footrest at the back of the carrier. The footrest then slides through the guides until the ends of the wire frame snap into the footrest locking points. These locking points may be slots or openings in the side plates of the carrier, located at the end of the guides. The footrest can be returned to the stored position by pushing both wire ends inward, freeing them from the locking points, and subsequent pushing the cross-piece of the wire frame toward the back plate of the shell, whereby the guides guide the footrest to the stored position. The guides could be retention ribs or grooves, situated on the inner surfaces of the side plates of the shell.
The shell of the portable carrier may further comprises a tubing guide in one side plate and the back plate. The tubing guide allows the tubing used with the carrier to be protectively routed around the shell towards the user. Thereby, the tubing guide minimizes the risk of tubing becoming kinked or entangled within a carrier bag.

The form and location of the feed pump holding element will depend upon the pump that is required to be retained within the shell. It will be understood that there can be considerable variation in such pumps. In one embodiment, the portable carrier may have the feed pump holding element extending from the bottom tray and side plates in the form of support ribs. A locking element and a power adapter opening may also be provided. The support ribs may be located at the lower front portion of the sidewalls whereby the pump can be supported ahead of the drip chamber. In this configuration the drip chamber and/or tubing connection is inaccessible from all sides, and thus fully protected. The support ribs and locking element ensure the pump remains in position throughout any carrier motions. If required, the pump can be connected to an external power source through the power adapter opening.

The feed pump holding element may include an opening or inlet in the bottom tray. This allows the pump to be more easily gripped or held in one hand on inserting the pump into the shell or on its subsequent removal, aiding access to the container and drip chamber/tubing connection. Although referred to as an opening, the feed pump holding element may alternatively be considered as extensions of the side plates that extend beyond the bottom tray and bottom tray.

The shell of the carrier may be sized to fit inside a backpack and sized to hold a range of known enteral food and medical liquid containers. The shape of the shell is then optimized for comfortable carrying inside a backpack, whilst suitable for holding a range of containers. The carrier may be designed to fit a specific backpack or a backpack may be designed around the carrier. The carrier may also be carried in a bag of choice from the user, the bag being a backpack or any other type of bag fitting around the carrier. Whilst being carried in a bag, the shell provides rigidity to the bag and helps to protect the pump and container from being squashed. To aid comfortable carrying, the weight of the carrier is minimized such that the weight of the carrier is kept between 300 grams and 500 grams.

The shell may be dimensioned to have a height of between 20 cm and 50 cm, preferably between 23 cm and 26 cm. The width between the side plates may be between 10 cm and 30 cm, preferably between 13 cm and 15 cm. The bottom tray may have a depth from front to back of between 10 cm and 30 cm, preferably between 12cm and 15 cm. The side plates may extend forwards from the back plate by from 15 cm to 30 cm at the base and may taper upwards to between 5 cm and 10 cm at their upper limit. The bottom tray may have a depth i.e. measured in the height direction of the shell, between 0 cm and 3 cm, preferably between 0.5 cm and 2 cm, and any dish-shaped region is preferably sized to largely match the depth and width of the bottom plate. It may have a volume of from 50 cc to 200 cc, preferably as large as possible within the space available in order to maximise its ability to collect spillage.
The position of the height of the shelf compared to the bottom of the shell may be dimensioned between 7 - 25cm, depending on the type of reservoir and feeding set.

According to another aspect, the shell of the portable carrier may be made of dishwasher proof material, preferably an amorphous copolyester such as TritanTM. As a result, the whole carrier can be simply and hygienically cleaned in the dishwasher without discoloration or the shell material becoming damaged. The shell and shelf could be made in an injection moulding process, allowing both parts to be integrally formed. Furthermore, the shell could be made transparent, allowing visual control during use.

The present invention is further aimed at an assembly, comprising a portable carrier as described above or hereinafter and a container holding enteral food or a liquid. The assembly may further comprise a feeding set with or without a drip chamber, a feed pump and/or a bag in which the aforementioned elements of the assembly are contained, wherein the bag has a base adapted to fit the bottom tray or wherein the bag is sized to snuggly receive the shell.

### Brief description of the drawings

The invention will be discussed in more detail below, with reference to the attached drawings in which illustrative embodiments thereof are shown. The drawings are intended exclusively for illustrative purposes and not as a restriction of the inventive concept. The scope of the invention is only limited by the definitions presented in the appended claims.
Figure 1 depicts a mobile enteral feed assembly including a portable carrier according to the invention.
Figure 2 shows an isometric view of the front side of the portable carrier of Figure 1.
Figure 3 shows an isometric view of the rear side of the portable carrier of Figure 2, with extended container stabilizer and footrest.
Figure 4a shows an isometric frontal view of a container rest shelf for a carrier according to the invention.
Figure 4b shows an isometric rear view of a container rest shelf for a carrier according to the invention.
Figure 5 shows an assembly of the portable carrier of Figure 2 and a feed pump.
Figure 6 shows an assembly of the portable carrier of Figure 2 with a container and feeding set.
Figure 7 shows an assembly of the portable carrier of Figure 2 with a container and feeding set including a drip chamber.
Figure 8 shows the assembly of Figure 7 with a feed pump.

### Description of embodiments

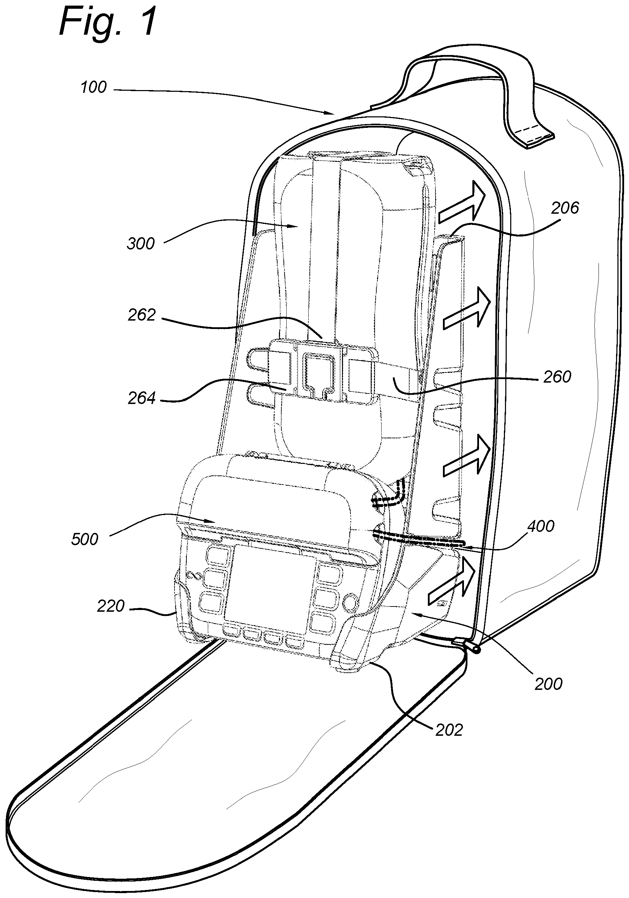

Figure 1 depicts a mobile enteral feed assembly 1 according to the invention. The assembly 1 comprises a backpack 100, a portable container carrier 200, an enteral feed or liquid container 300, feeding set 400 and a feed pump 500.

The enteral feed or liquid container 300 is connected to the feeding set 400 at its main opening and placed upside-down into the carrier 200. Due to the upside-down placement of the container 300, the enteral feed or liquid will naturally flow into the tubing 400 under gravity. The carrier is designed for offering good stability to the container and equipped with fastening means which hold the container in a predetermined position within the carrier.

The feeding set 400 is run through the feed pump 500, wherein the pump is set up to pump the enteral food or liquid from the container through the feeding set 400 at a predetermined rate towards a patient carrying the assembly. The feed pump 500 may be any conventional feed pump such as the Flocare Infinity TM pump available from Nutricia and is not further part of the present invention.

The feed pump 500 is located in a pump holding element, which is a part of the portable carrier 200. The carrier 200 forms a protective shell, partially surrounding the container and the feed pump. Furthermore, the carrier 200 is shaped to fit into a backpack 100, such that the back plate of the carrier ergonomically sits against the back of a person carrying the backpack 100 and the bottom tray of the carrier keeps the carrier stable within the bag, while preferably also serving as a spillage tray, keeping the bag clean.

The backpack 100 shown in this example is a backpack specifically designed to hold the carrier 200 with the container and feed pump. This specifically designed backpack illustrated includes a large access zip for accessing, inserting and removing the entire carrier and a smaller access zip for accessing the control panel of the feed pump. Alternatively, the backpack could be any bag chosen by the patient.

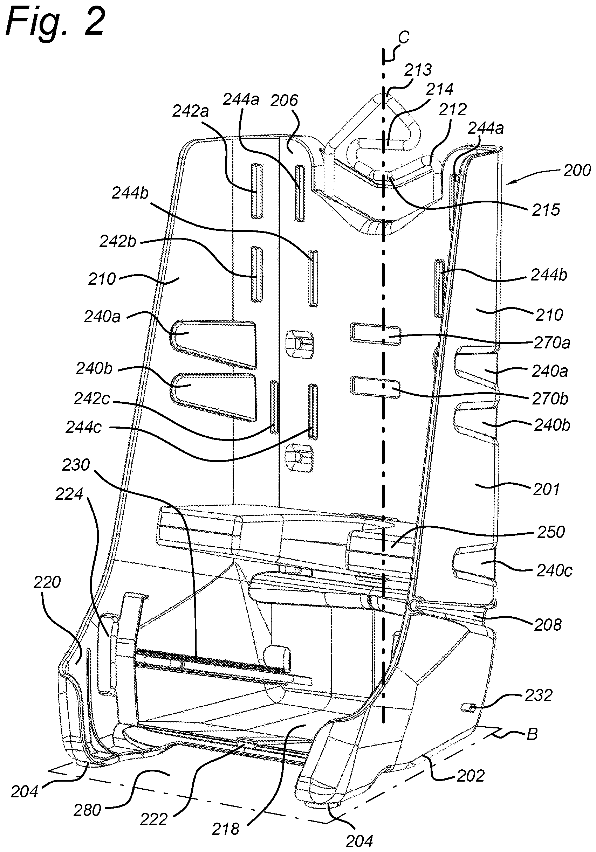

More details of the portable carrier are explained in reference to Figures 2 - 4.

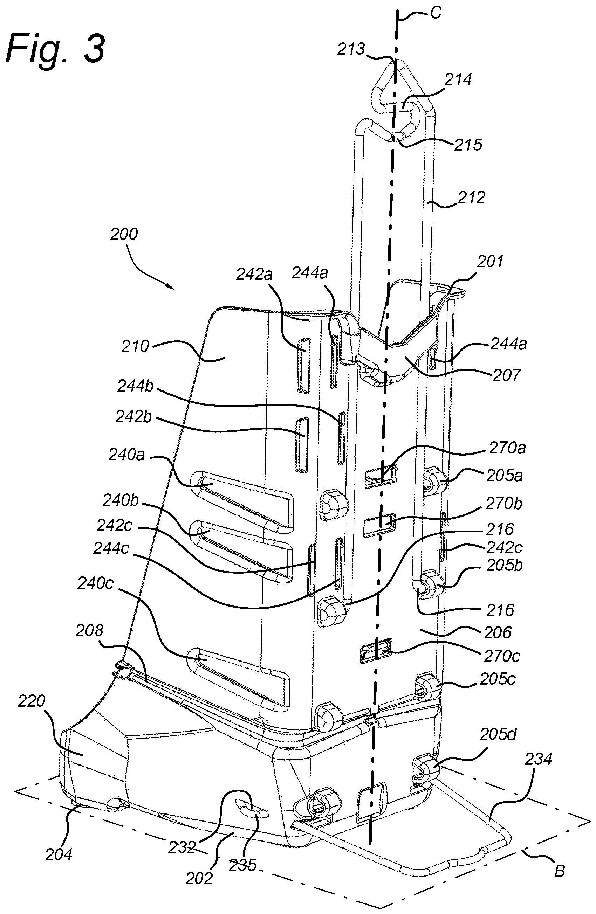

Figures 2 and 3 show isometric views of the front and back of a portable carrier 200 according to the invention. The carrier comprises a shell 201 with an integrated feed pump holding element 220 and a repositionable container shelf 50, whereby the shell is further equipped with an adjustable container stabilizer 212 and an adjustable footrest 234. The shell 201 has a bottom tray 202, a back plate 206 and two side plates 210.

The shell's bottom tray 202 is shaped to form a stable base to the carrier 200, both when carried in a backpack 100 and when used as a table top stand. To this end, the front and rear corner areas of the bottom tray are shaped to rest within the same plane B, whereby at least the front bottom tray corners contain footrests 204. Furthermore, the bottom tray 202 includes a spillage tray 218 for collecting any enteral food or liquid which may be spilled from the connection between the container 300 and the feeding set 400, thus keeping the backpack 100 or table on which the carrier is used clean.

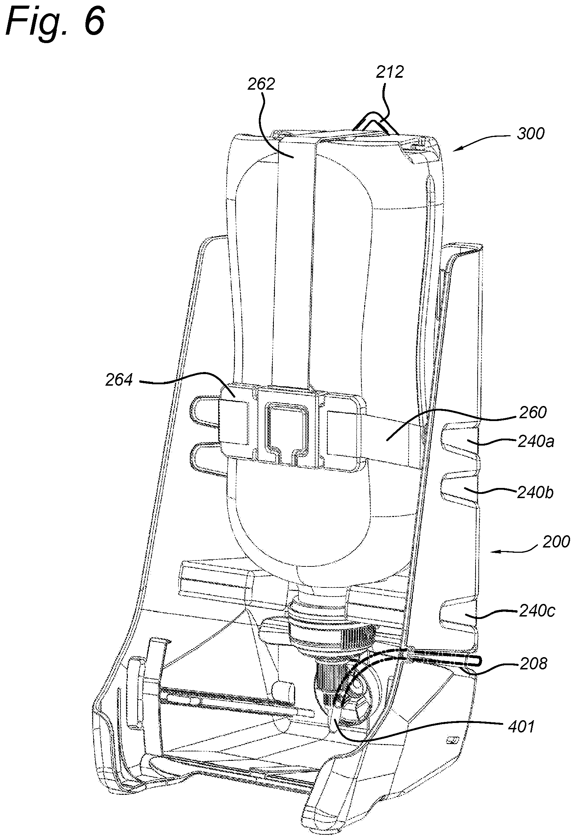

The shell's back plate 206 extends substantially perpendicular to the bottom tray 202 from the back edge of the bottom tray 202. The back plate 206 comprises wide strap openings 242 and narrow strap openings 244, shelf locking openings 270 and stabilizer locking elements 205. The strap openings 242, 244 form vertical slots with respect to the bottom tray 202, which openings are provided for slotting through container holding straps (see Figure 6). The wide strap openings 242 are set at a wider distance around a centreline C of the back plate 206 than the narrow strap openings 244, allowing for tight strapping of both wide and narrow containers. This makes the container carrier also suitable for use with, for example, baby-bottles. The wide strap openings 242 include three pairs of strap openings 242 a-c set at three different heights with respect to the bottom tray 202, which allow the strap to be placed according to the height of the container. The same applies to the narrow strap openings 244 a-c. It will be understood that alternative numbers and distributions of slots or other alternative attachment provisions may be provided.

The shelf locking openings 270 a-c in this example are provided as a row of horizontal slots with respect to the bottom tray 202, placed symmetrically on the centreline C of the back plate. The shelf locking openings 270 a-c are set at three different heights with respect to the bottom tray 202. The height at which each opening 70 a, b, c is placed corresponds to a predetermined height at which a container 300 can be placed into the carrier 200. The stabilizer locking elements 205 are described in more detail in relation to the adjustable stabilizer element 212.

The side plates 210 of the shell 201 extend substantially perpendicular to the bottom tray 202 and to the back plate 206. The side plates 210 have a width which is large enough to cover the depth of most used enteral food and other liquid containing containers. The shell 201 is triangular/trapezoidal in side view. As a result, the shell 201 provides substantial protection to the containers whilst being carried in a bag, while still allowing good access to the container.

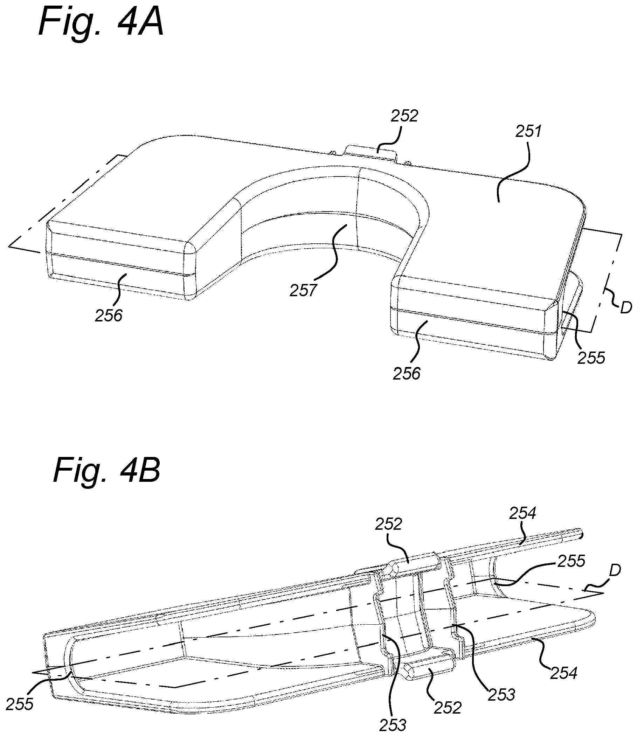

Further, the side plates 210 each comprise table stand ribs 240, a guiding rib 230 and a locking opening 232. The guiding rib 230 and locking opening 232 are described in more detail in relation to the adjustable food rest 234 below. The table stand ribs 240 a-c are placed in pairs divided over the two side plates 210, such that the side plates 210 mirror each other with respect to centreline C, for supporting a container rest shelf 250 (described in more detail in the figure description for Figures 4A and 4B). The ribs 240 a-c are shaped to match with the shape of the container rest shelf 250, whereby the shelf 250 contributes positively to the stiffness of the shell 201. Additionally, the ribs 240 a-c are placed such that the container rest shelf 250 is fitted at an angle of at least 5 degrees compared to the bottom tray 202, allowing the container to rest naturally against the back plate 206. Furthermore, the table stand ribs 240 a, b, c are located at three different heights with respect to the bottom tray, allowing the shelf 250 to be placed at three different heights. These heights are predetermined such that the carrier 200 remains as compact as possible, whilst providing sufficient space between the shelf 250 and the bottom tray 202 such that no kinks occur in the feeding set 400. A first pair of ribs 240c is placed at the lowest height, for use of a container with a feeding set without a drip chamber. A second pair of ribs 240b is set at an intermediate height, for use of a container with a feeding set 400 with a drip chamber of relative small size. A third pair of ribs 240a is set at the largest height, for use of a container with a feeding set with a large size drip chamber. The aforementioned openings 270 a-c in the back plate 206 are matched to the height of the pairs of ribs 240 a-c.

A tube guiding element 208 is formed in one side plate 210 and the back plate 206. The tube guiding element 208 provides a protected route for tubing to run through, towards the patient and reduces the risk of the tubing kinking inside the backpack.

The feed pump holding element 220 is formed as part of the shell, wherein the side plates 210 and bottom tray 202 are extended to form a sleeve into which the feed pump 500 easily fits. One side plate 210 contains an opening 224 in the feed pump holding element 220, through which a power adapter may be connected to the feed pump 500, for providing power supply while the feed pump 500 is installed in the shell 201. Furthermore, the region of the shell 201 below the feed pump has an inlet 280, allowing a user good access to the pump 500 during installation and removal from the shell 201. Additionally, the bottom tray 202 is equipped with a locking element 222, allowing the feed pump to be locked inside the holding element 220.

The shell 201 is formed as a single piece, manufactured in one injection moulding step of a rigid and transparent, amorphous copolyester material available as TritanTM from Eastman corp. It is dishwasher proof and can thus be easily washed after removal from the bag 100. It will be understood that other plastic materials may be used, including both rigid and semi rigid plastics.

As previously listed, the portable carrier 200 depicted in Figures 2 and 3 further comprises an adjustable stabilizer element 212. The stabilizer element 212 is a longitudinal element, comprising a hanging hook 213, a horizontal section 214 and a holding strap connection bar 215 on one end which extends out from the top edge of the back plate 206, and connector elements 216 on the other end. In the present example, the stabilizer element 212 is a wire frame, shaped from a single piece of wire, whereby the wire ends form the connector elements 216. The stabilizer element 212 is located at or (partially) in the back plate 206 of the shell 201, such that a longitudinal centreline of the stabilizer substantially coincides with a longitudinal centreline C of the back plate 206. The stabilizer element 212 is installed in/onto the back plate 206 such that the stabilizer element 212 is movable only along the centreline C, between a first position wherein the stabilizer element 212 is mostly hidden in/behind the back plate 206 with only the hook 213, horizontal section 214 and holding strap connection bar 215 extending out above the top edge of the back plate 206, and a second position in which the stabilizer element 212 mostly extends above the top edge of the back plate 206, such that only the bottom end comprising the connector elements 216 remains in/behind the back plate 206. To ensure the alignment of the stabilizer element 212 and the back plate 206, and to further prevent any rotation of the stabilizer element 212 with respect to the back plate 206, the upper part of the back plate comprises a stabilizer rib 207. This stabilizer rib 207 is integrally formed with the shell 201 and lies in a plane parallel to the back plate 206, such that the back plate 206 is at a first side of the stabilizer element 212 and the stabilizer rib 207 is at a second, opposing side of the stabilizer element 212. The stabilizer element 212 is thus partially surrounded and enclosed by the stabilizer rib 207. Further, the stabilizer element 212 can be locked in position at at least two different heights, using the previously mentioned stabilizer locking elements 205 a-d, located in the back plate 206. In the present example, the back plate 206 is supplied with four pairs of locking elements 205 a-d, placed symmetrically around the centreline C of the plate. The locking elements 205 a- d are set at four different heights with respect to the bottom tray 202, allowing the stabilizer element 212 to be locked at four different extension levels with respect to the back plate 206.

The higher locking positions 205 a, b effectively increase the supported height provided by the carrier 200, and can be used to stabilize large containers, which may be larger than 500 ml. Furthermore, the stabilizer element 212 provides additional utility in that hook 213 may be used further as carrier hand grip or for hanging containers from. Finally, due to the smooth and continuous shape of the wire, the stabilizer element 212 does not have any sharp and/or protruding ends, the stabilizer element is non-damaging to the carrier bag and safe for the user to walk around with.

Lastly, the portable carrier 200 comprises the previously referenced footrest 234. In the present example, the footrest 234 is a bracket shaped wire, which is shaped to match a cross-sectional shape of the shell 201. The footrest 234 is spaced from the bottom plane B and set at a downward angle towards the back plate 206. The ends of the bracket shaped wire are shaped to form locking elements 235.

The footrest 234 is positioned inside a bottom section of the shell 201, below the lowest position of the container support shelf 250, such that the footrest 234 is slidably deployable from the back of the shell 201. In the stored position, the footrest 234 is located entirely within the perimeter of the shell 201, whereby the two extending legs of the bracket shaped wire 234 are positioned within the shell 201, located in footrest guiding ribs 230 which are located in the side plates 210 and angled downward towards the back plate 206. When deploying the footrest 234 to its extended position, the legs of the bracket slide through the footrest guiding ribs 230, until reaching a final locked position, wherein the locking elements 235 snap into locking holes 232. Each side plate 210 comprises a locking hole 232 at the end of the footrest guiding rib 230 near the back plate 206. In this extended and locked position, the position of the footrest 234 with respect to the shell 201 is such that the back plate matching section of the bracket shaped wire rests in bottom plane B. The footrest 234 can be returned to the retracted position by pushing the locking elements 235 back into the locking holes 232 and sliding the bracket back inwards.

By extending the footrest 234, the footprint of the carrier 200 is increased, which further improves the stability of the carrier 200. This further improved stability allows the carrier 200 to be used safely as a table stand with heavier and larger containers filled with enteral food or liquid, which otherwise would cause the stand to become unstable due to having a higher centre of gravity.

Figures 4A and 4B show isometric front and rear views of the container rest shelf 250. The container rest shelf 250 has a front edge 256 with a recess 257, rear edges 254, side edges 255, a container rest surface 251 and a locking element comprising broad lip locks 252 and stability ribs 253. The container rest shelf 250 is symmetric with respect to a plane D, which extends from and is perpendicular to the front edge 256 between the broad lip locks 252. As a result, the shelf fits into the carrier, regardless if the presently visible resting surface 251 is facing up or down. Furthermore, the side edges 255 are shaped to fit around the table stand ribs 240 such that the ribs add stiffness to the rest shelf 250.

When fully installed over a pair of table stand ribs 240, the upper broad lip lock 252 is locked into a matching shelf locking opening 270. The stability ribs 253 add stiffness and stability to the back of the shelf 250, such that the broad lip lock 252 is prevented from bending under pressure from a container resting on the top surface 251. Due to the angle of at least 5 degrees at which the table stand ribs 240 are placed, the bottom lip lock 252 sits adjacent to the back plate 206, when the shelf 250 is locked in position in the shell 201. The container rest shelf 250 can be made from the same material and using the same manufacturing method as the shell 201. The shelf 250 is also preferably made of a dishwasher proof material.

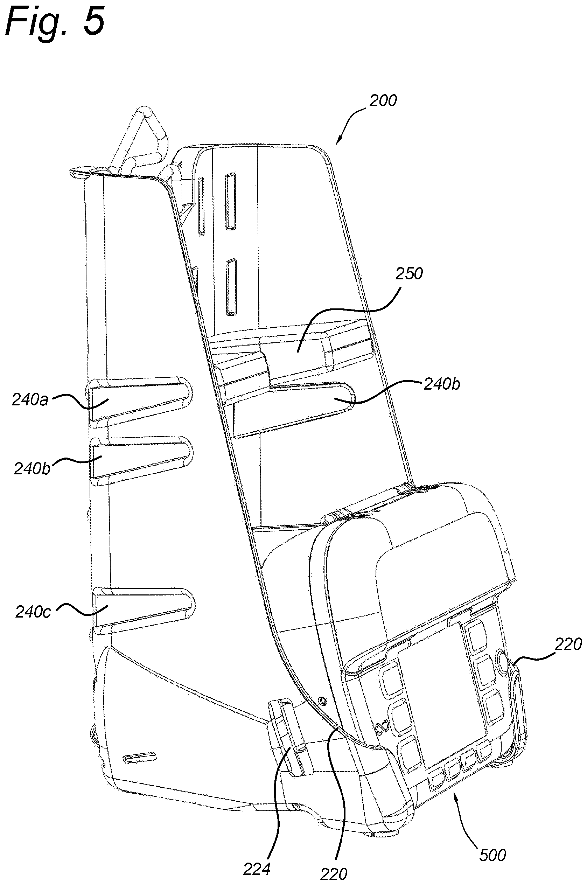

Figure 5 shows an assembly of a portable carrier 200 according to the invention and a feed pump 500. The feed pump 500 is held by the container carrier 200 in the feed pump holding element 220. The opening 224 in the side plate of the shell, allowing a power adapter to be used for powering the feed pump during use of the pump 500 in the carrier 200, can be clearly seen. The container support shelf 250 is located at the highest table stand ribs 240a.

Figure 6 shows an assembly of a container 300 and a carrier 200 according to the invention, whereby the container 300 is attached to tubing 401, without a drip chamber. The container support shelf 250 is located at the lowest table stand ribs 240c, resulting in a compact assembly with sufficient space below the container 300 for the tubing 401 to run without any kinks in the space below the container 300 from the container to the tubing guide 208.

Container straps 260, 262 have been added, for holding the container 300 tightly in the carrier, ensuring the container does not move out of position within the carrier during any use. Horizontal strap 260 runs through an strap slots 242b in the back plate (not visible), chosen based on container dimensions and shelf height. Vertical strap 262 is attached to the stabilizer element 212 (attachment not visible) on one end. The free ends of the straps 260, 262 are joined together through a joining element 264, such that the straps are further adjustable to tightly fit around any container placed therein.

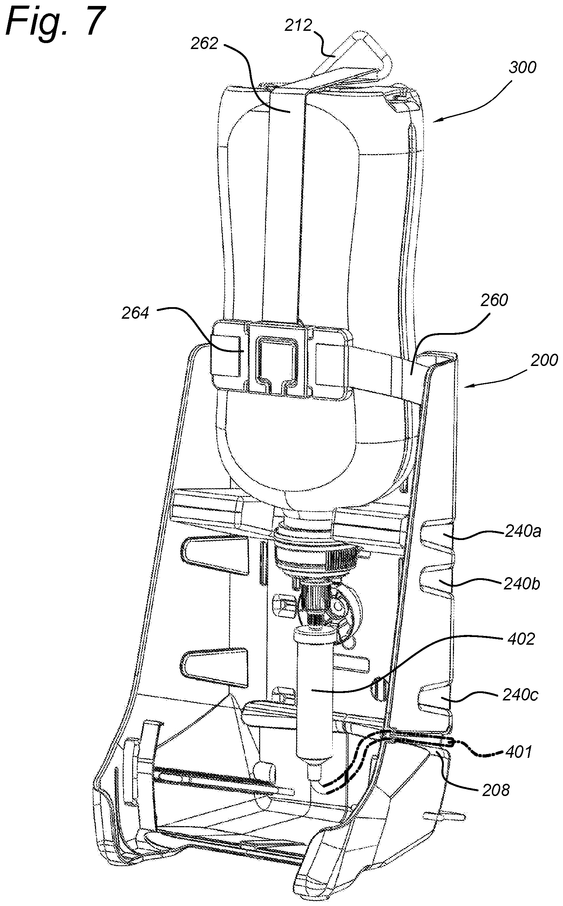

Figure 7 shows an assembly of a container 300, feeding set 400 comprising tubing 401 with a drip chamber 402 and a carrier 200 according to the invention. To provide the required space for fitting the feeding set 400 with drip chamber 402 below the container 300 without suffering kinks in the tubing 401, the rest shelf 250 is positioned on the highest table stand ribs 240a. Additionally, the stabilizer element 212 is locked in a higher position, providing the higher placed container 300 with sufficient support. Further, the container straps 260, 262 are relocated compared to Figure 6, to match the shelf height of figure 7. To this end, the horizontal strap 260 is run through the highest located pair of strap slots 242a in the back plate (not visible). The vertical strap 262, which is attached to the stabilizer element 212, has moved up together with this element, and thus does not require any further adjustment.

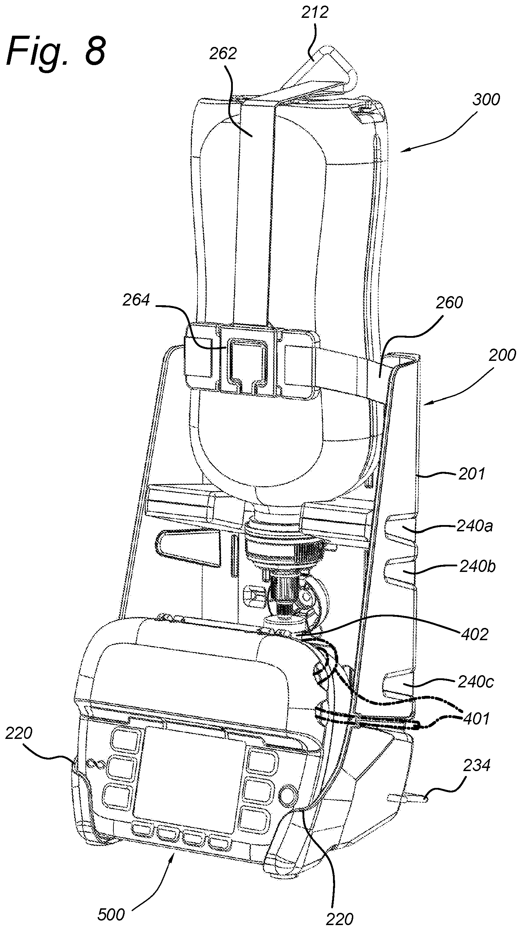

Figure 8 shows an assembly 1 of a container 300, feeding set 400 with a drip chamber 402, a carrier 200 and a feed pump 500. The set-up of this assembly is similar to the set-up shown in figure 7, but has the feed pump 500 inserted into the feed pump holding element 220 of the shell 201.

Further obvious modifications and alterations will occur to others upon reading and understanding the preceding detailed description. It is intended that the invention be construed as including all such modifications and alterations insofar as they come within the scope of the appended claims.

## Claims

1. A portable carrier (200) for transporting and administering enteral food or other liquids provided in a container (300), the carrier comprising
a semi-rigid or rigid shell (201), comprising a bottom tray (202), a back plate (206), two side plates (210) and a feed pump holding element (220), wherein the feed pump holding element comprises extensions of the side plates that extend beyond the bottom tray and the bottom tray is shaped to form a stable base to the carrier, the shell further being shaped to fit into a backpack (100), such that the back plate sits against the back of a person carrying the backpack;
support elements for holding the container in place within the shell, the support elements comprising a lower support element for location beneath the container, a rear support element for engaging a rear surface of the container and a container retaining element for retaining the container in position within the shell, wherein the support elements are height adjustable with respect to the bottom tray, thereby allowing a container to be supported at different positions above the bottom tray and wherein the rear support element comprises an adjustable container stabilizer (212), extendable vertically with respect to the back plate.

2. The portable carrier (200) according to claim 1, wherein the bottom tray (202) is a spillage tray (218).

3. The portable carrier (200) according to claim 1 or 2, wherein the lower support element comprises a container rest shelf (250), extending between the side plates (210), which is repositionable along the back plate (206), such that a distance between the shelf and the bottom tray (202) is variable, wherein the side plates preferably each comprise two or more ribs (240), provided in pairs at two or more heights from the bottom tray, for positioning of the shelf.

4. The portable carrier (200) according to claim 3, wherein the shelf (250) comprises one or more locking elements (205) at a back edge, and wherein the back plate (206) of the shell (201) comprises one or more locking element receiving slots (242, 244) located at two or more heights from the bottom tray (202) for receiving the one or more locking elements.

5. The portable carrier (200) according to claims 3 or 4, wherein the shelf (250) is slidably positionable over each set of ribs (240), whereby the ribs extend in a direction forwards from the back plate (206) and are tapered in this direction.

6. The portable carrier (200) according to any one of the previous claims, wherein the adjustable container stabilizer (212) is extendable to a position at which a centre of gravity of the container (300) extends above the shell (201) of the carrier.

7. The portable carrier (200) according to any preceding claim, wherein the stabilizer (212) is located within the back plate (206) such that a longitudinal centreline of the stabilizer is parallel with a centreline (C) of the back plate, whereby the stabilizer is slidably movable along the centreline, and wherein the back plate further comprises container stabilizer locking elements (205), located at two or more heights from the bottom tray (202), preferably wherein the container stabilizer locking elements are spaced in pairs, symmetrically around the centreline of the back plate and located at two or more heights from the bottom tray.

8. The portable carrier (200) according to any preceding claim, wherein the container stabilizer (212) comprises a hanging hook (213) and/or wherein the adjustable container stabilizer is a wire frame, formed out of a single continuous wire of which the wire ends are lockable in the stabilizer locking elements (205).

9. The portable carrier (200) according to any one of the previous claims, wherein the back plate (206) of the shell (201) comprises a first set of strap slots (242) and a second set of strap slots (244), the sets being spaced in pairs around and along the centreline (C) of the back plate, and wherein a distance between the centreline and the first set of strap slots (242) is larger than a distance between the centreline and the second set of strap slots (244); and the container retaining element comprises at least one strap (260), connectable through a pair of strap slots, for encircling the container (300), preferably wherein the container retaining element further comprises at least one vertical strap (262), which is attached to the adjustable container stabilizer, and wherein the straps are adaptable to the size and shape of the container (300).

10. The portable carrier (200) according to any one of the previous claims, the carrier further comprising an adjustable footrest (234), the footrest being adjustable between a first position, wherein the footrest is within a perimeter of the shell (201), and a second position, wherein the footrest extends rearwardly out from the shell, to a position where it can engage a surface on which the shell is placed, to stabilise the carrier, preferably wherein the footrest comprises a wire frame having dimensions corresponding to a horizontal cross-section of the shell above the bottom tray (202), and more preferably wherein the shell in addition further comprises guides (230) within which the wire frame can slide and footrest locking points (232) into which ends (235) of the wire frame can locate.

11. The portable carrier (200) according to any one of the previous claims, wherein the shell (201)
- is sized to hold a range of known enteral food and medical liquid containers (300) and/or
- made of dishwasher proof material and/or
- further comprises a tubing guide (208) in one side plate (210) and the back plate (206).

12. The portable carrier (200) according to any one of the previous claims, wherein the feed pump holding element (220) comprises support ribs, a locking element (222) and/or a power adapter opening (224), preferably wherein the feed pump holding element includes an inlet (280) in the bottom tray (202) at a position below the pump.

13. An assembly (1), comprising
the portable carrier (200) according to any one of the previous claims; and
a container (300) holding enteral food or a liquid.

14. Assembly according to claim 13, further comprising a feeding set (400) with or without a drip chamber (402) and/or further comprising a feed pump (500).

15. Assembly according to claim 13 or 14, further comprising a bag (100) which contains the elements listed in any one of claims 13 or 14, wherein the bag has a base adapted to fit the bottom tray and/or wherein the bag is sized to snuggly receive the shell.

## Patentansprüche

1. Tragbarer Träger (200) zum Transportieren und Verabreichen enteraler Nahrung oder anderer Flüssigkeiten, die in einem Behälter (300) bereitgestellt sind, wobei der Träger Folgendes umfasst:
eine halbstarre oder starre Schale (201), umfassend eine Bodenschale (202), eine Rückplatte (206), zwei Seitenplatten (210) und ein Förderpumpenhalteelement (220); wobei das Förderpumpenhalteelement Verlängerungen der Seitenplatten umfasst, die sich über die Bodenschale hinaus erstrecken, und die Bodenschale so geformt ist, dass sie eine stabile Basis für den Träger bildet, wobei die Schale ferner so geformt ist, dass sie in einen Rucksack (100) passt, so dass die Rückplatte am Rücken einer Person anliegt, die den Rucksack trägt;
Stützelemente zum Halten des Behälters an seinem Platz in der Schale, wobei die Stützelemente ein unteres Stützelement zur Anordnung unter dem Behälter, ein hinteres Stützelement zum Anliegen an eine Rückwand des Behälters und ein Behälterhalteelement zum Halten des Behälters in Position innerhalb der Schale umfassen, wobei die Stützelemente in Bezug auf die Bodenschale höhenverstellbar sind, wodurch ein Behälter an verschiedenen Positionen über der Bodenschale gestützt werden kann und wobei das hintere Stützelement einen verstellbaren Behälterstabilisator (212) umfasst, der vertikal in Bezug auf die Rückplatte ausfahrbar ist.

2. Tragbarer Träger (200) nach Anspruch 1, wobei die Bodenschale (202) eine Auslaufschale (218) ist.

3. Tragbarer Träger (200) nach Anspruch 1 oder 2, wobei das untere Stützelement eine sich zwischen den Seitenplatten (210) erstreckende Behälterablage (250) umfasst, die entlang der Rückplatte (206) neu positionierbar ist, so dass ein Abstand zwischen der Ablage und der Bodenschale (202) variabel ist, wobei die Seitenplatten vorzugsweise jeweils zwei oder mehr Rippen (240) umfassen, die paarweise in zwei oder mehr Höhen von der Bodenschale vorgesehen sind, um die Ablage zu positionieren.

4. Tragbarer Träger (200) nach Anspruch 3, wobei die Ablage (250) ein oder mehrere Verriegelungselemente (205) an einer hinteren Kante umfasst, und wobei die Rückplatte (206) der Schale (201) einen oder mehrere Verriegelungselement-Aufnahmeschlitze (242, 244) umfasst, die sich in zwei oder mehr Höhen von der Bodenschale (202) befinden, um das eine oder die mehreren Verriegelungselemente aufzunehmen.

5. Tragbarer Träger (200) nach Anspruch 3 oder 4, wobei die Ablage (250) über jeden Satz von Rippen (240) gleitend positionierbar ist, wobei sich die Rippen von der Rückplatte (206) aus in eine Richtung nach vorne erstrecken und in dieser Richtung verjüngt sind.

6. Tragbarer Träger (200) nach einem der voranstehenden Ansprüche, wobei der verstellbare Behälterstabilisator (212) in eine Position ausfahrbar ist, in der ein Schwerpunkt des Behälters (300) über die Schale (201) des Trägers hinausragt.

7. Tragbarer Träger (200) nach einem der voranstehenden Ansprüche, wobei der Stabilisator (212) innerhalb der Rückplatte (206) angeordnet ist, so dass eine Längsmittellinie des Stabilisators parallel zu einer Mittellinie (C) der Rückplatte verläuft, wodurch der Stabilisator entlang der Mittellinie verschiebbar ist, und wobei die Rückplatte ferner Behälterstabilisator-Verriegelungselemente (205) umfasst, die in zwei oder mehr Höhen von der Bodenschale (202) angeordnet sind, wobei die Behälterstabilisator-Verriegelungselemente vorzugsweise paarweise beabstandet sind, symmetrisch um die Mittellinie der Rückplatte herum und in zwei oder mehr Höhen von der Bodenschale angeordnet sind.

8. Tragbarer Träger (200) nach einem der voranstehenden Ansprüche, wobei der Behälterstabilisator (212) einen Aufhängehaken (213) umfasst und/oder wobei der verstellbare Behälterstabilisator ein Drahtgestell ist, das aus einem einzigen Endlosdraht gebildet ist, dessen Drahtenden in den Stabilisatorverriegelungs-elementen (205) verriegelbar sind.

9. Tragbarer Träger (200) nach einem der voranstehenden Ansprüche, wobei die Rückplatte (206) der Schale (201) einen ersten Satz von Gurtschlitzen (242) und einen zweiten Satz von Gurtschlitzen (244) umfasst, wobei die Sätze paarweise um und entlang der Mittellinie (C) der Rückplatte beabstandet sind, und wobei ein Abstand zwischen der Mittellinie und dem ersten Satz von Gurtschlitzen (242) größer ist als ein Abstand zwischen der Mittellinie und dem zweiten Satz von Gurtschlitzen (244); und das Behälterhalteelement mindestens einen Gurt (260) umfasst, der durch ein Paar von Gurtschlitzen verbindbar ist, um den Behälter (300) zu umschließen, wobei das Behälterhalteelement vorzugsweise ferner mindestens einen vertikalen Gurt (262) umfasst, der an dem verstellbaren Behälterstabilisator befestigt ist, und wobei die Gurte an die Größe und Form des Behälters (300) anpassbar sind.

10. Tragbarer Träger (200) nach einem der voranstehenden Ansprüche, wobei der Träger ferner eine einstellbare Fußstütze (234) umfasst, wobei die Fußstütze zwischen einer ersten Position, in der sich die Fußstütze innerhalb eines Umfangs der Schale (201) befindet, und einer zweiten Position einstellbar ist, in der sich die Fußstütze nach hinten aus der Schale heraus in eine Position erstreckt, in der sie mit einer Oberfläche in Eingriff kommen kann, auf die die Schale gestellt wird, um den Träger zu stabilisieren, wobei die Fußstütze vorzugsweise einen Drahtrahmen umfasst, der Abmessungen aufweist, die einem horizontalen Querschnitt der Schale oberhalb der Boden schale (202) entsprechen, und wobei die Schale außerdem vorzugsweise Führungen (230), in denen der Drahtrahmen gleiten kann, und Fußstützenverriegelungspunkte (232) umfasst, in die Enden (235) des Drahtrahmens eingreifen können.

11. Tragbarer Träger (200) nach einem der voranstehenden Ansprüche, wobei die Schale (201)
- so bemessen ist, dass sie eine Reihe von bekannten enteralen Nahrungs- und medizinischen Flüssigkeitsbehältern (300) aufnehmen kann und/oder
- aus spülmaschinenfestem Material gefertigt ist und/oder
- ferner eine Schlauchführung (208) in einer Seitenplatte (210) und der Rückplatte (206) aufweist.

12. Tragbarer Träger (200) nach einem der voranstehenden Ansprüche, wobei das Halteelement (220) für die Förderpumpe Stützrippen, ein Verriegelungselement (222) und/oder eine Stromadapteröffnung (224) aufweist, wobei das Halteelement für die Förderpumpe vorzugsweise einen Einlass (280) in der Bodenschale (202) an einer Position unterhalb der Pumpe aufweist.

13. Anordnung (1), umfassend
den tragbaren Träger (200) nach einem der voranstehenden Ansprüche; und
einen Behälter (300), der enterale Nahrung oder eine Flüssigkeit enthält.

14. Anordnung nach Anspruch 13, ferner umfassend ein Nahrungsset (400) mit oder ohne eine Tropfkammer (402) und/oder ferner umfassend eine Förderpumpe (500).

15. Anordnung nach Anspruch 13 oder 14, die ferner eine Tasche (100) umfasst, die die in einem der Ansprüche 13 oder 14 aufgeführten Elemente enthält, wobei die Tasche einen Boden aufweist, der so angepasst ist, dass er in die Bodenschale passt, und/oder wobei die Tasche so bemessen ist, dass sie die Schale eng anliegend aufnimmt.

## Revendications

1. Support portable (200) permettant de transporter et d'administrer des aliments entéraux ou d'autres liquides contenus dans un contenant (300), le support comprenant
une coque semi-rigide ou rigide (201), comprenant un plateau inférieur (202), une plaque arrière (206), deux plaques latérales (210) et un élément de maintien de pompe d'alimentation (220), dans lequel l'élément de maintien de pompe d'alimentation comprend des extensions des plaques latérales qui s'étendent au-delà du plateau inférieur et le plateau inférieur est conçu pour former une base stable pour le support, la coque étant en outre mise en forme pour s'ajuster dans un sac à dos (100), de telle sorte que la plaque arrière repose contre le dos d'une personne portant le sac à dos ;
des éléments de support pour maintenir le contenant en place à l'intérieur de la coque, les éléments de support comprenant un élément de support inférieur pour un positionnement sous le contenant, un élément de support arrière pour une mise en prise avec une surface arrière du contenant et un élément de retenue de contenant pour retenir le contenant en position à l'intérieur de la coque, dans lequel les éléments de support sont ajustables en hauteur par rapport au plateau inférieur, en permettant ainsi à un contenant d'être supporté à différentes positions au-dessus du plateau inférieur et dans lequel l'élément de support arrière comprend un stabilisateur de contenant ajustable (212), extensible verticalement par rapport à la plaque arrière.

2. Support portable (200) selon la revendication 1, dans lequel le plateau inférieur (202) est un plateau de déversement (218).

3. Support portable (200) selon la revendication 1 ou 2, dans lequel l'élément de support inférieur comprend une étagère de support de contenant (250), s'étendant entre les plaques latérales (210), qui peut être repositionnée le long de la plaque arrière (206), de telle sorte qu'une distance entre l'étagère et le plateau inférieur (202) est variable, dans lequel les plaques latérales comprennent de préférence chacune deux nervures (240) ou plus, prévues par paires à deux hauteurs ou plus du plateau inférieur, pour un positionnement de l'étagère.

4. Support portable (200) selon la revendication 3, dans lequel l'étagère (250) comprend un ou plusieurs éléments de verrouillage (205) au niveau d'un bord arrière, et dans lequel la plaque arrière (206) de la coque (201) comprend une ou plusieurs fentes de réception d'élément de verrouillage (242, 244) situées à deux hauteurs ou plus à partir du plateau inférieur (202) pour recevoir les un ou plusieurs éléments de verrouillage.

5. Support portable (200) selon la revendication 3 ou 4, dans lequel l'étagère (250) peut être positionnée de manière coulissante sur chaque ensemble de nervures (240), dans lequel les nervures s'étendent dans une direction vers l'avant à partir de la plaque arrière (206) et sont effilées dans cette direction.

6. Support portable (200) selon l'une quelconque des revendications précédentes, dans lequel le stabilisateur de contenant ajustable (212) peut être étendu vers une position dans laquelle un centre de gravité du contenant (300) s'étend au-dessus de la coque (201) du support.

7. Support portable (200) selon l'une quelconque des revendications précédentes, dans lequel le stabilisateur (212) est situé à l'intérieur de la plaque arrière (206) de telle sorte qu'une ligne médiane longitudinale du stabilisateur est parallèle à une ligne médiane (C) de la plaque arrière, de telle sorte que le stabilisateur est mobile de manière coulissante le long de la ligne médiane, et dans lequel la plaque arrière comprend en outre des éléments de verrouillage de stabilisateur de contenant (205), situés à deux hauteurs ou plus à partir du plateau inférieur (202), de préférence dans lequel les éléments de verrouillage de stabilisateur de contenant sont espacés par paires, de manière symétrique autour de la ligne médiane de la plaque arrière et situés à deux hauteurs ou plus à partir du plateau inférieur.

8. Support portable (200) selon l'une quelconque des revendications précédentes, dans lequel le stabilisateur de contenant (212) comprend un crochet d'accrochage (213) et/ou dans lequel le stabilisateur de contenant ajustable est un fil de fer, formé à partir d'un fil continu unique dont les extrémités de fil métallique peuvent être verrouillées dans les éléments de verrouillage de stabilisateur (205).

9. Support portable (200) selon l'une quelconque des revendications précédentes, dans lequel la plaque arrière (206) de la coque (201) comprend un premier ensemble de fentes de sangle (242) et un second ensemble de fentes de sangle (244), les ensembles étant espacés par paires autour et le long de la ligne médiane (C) de la plaque arrière, et dans lequel une distance entre la ligne médiane et le premier ensemble de fentes de sangle (242) est supérieure à une distance entre la ligne médiane et le second ensemble de fentes de sangle (244) ; et l'élément de retenue de contenant comprend au moins une sangle (260), pouvant être connectée à travers une paire de fentes de sangle, pour encercler le contenant (300), de préférence dans lequel l'élément de retenue de contenant comprend en outre au moins une sangle verticale (262), qui est fixée au stabilisateur de contenant ajustable, et dans lequel les sangles peuvent être adaptées à la taille et à la forme du contenant (300).

10. Support portable (200) selon l'une quelconque des revendications précédentes, le support comprenant en outre un repose-pieds ajustable (234), le repose-pieds étant ajustable entre une première position, dans lequel le repose-pieds est à l'intérieur d'un périmètre de la coque (201), et une seconde position, dans lequel le repose-pieds s'étend vers l'arrière hors de la coque, jusqu'à une position dans laquelle il peut venir en prise avec une surface sur laquelle la coque est placée, pour stabiliser le support, de préférence dans lequel le repose-pieds comprend un cadre en fil métallique présentant des dimensions correspondant à une section transversale horizontale de la coque au-dessus du plateau inférieur (202), et de manière plus préférée dans lequel la coque comprend en outre des guides (230) à l'intérieur desquels le fil de fer peut coulisser et des points de verrouillage de repose-pieds (232) dans lesquels des extrémités (235) du fil de fer peuvent se positionner.

11. Support portable (200) selon l'une quelconque des revendications précédentes, dans lequel la coque (201)
- est dimensionnée pour contenir une gamme de contenants (300) d'aliments entéraux et de liquides médicaux connus et/ou
- est réalisée en un matériau résistant au lave-vaisselle et/ou
- comprend en outre un guide de tube (208) dans une plaque latérale (210) et la plaque arrière (206).

12. Support portable (200) selon l'une quelconque des revendications précédentes, dans lequel l'élément de maintien de pompe d'alimentation (220) comprend des nervures de support, un élément de verrouillage (222) et/ou une ouverture d'adaptateur d'alimentation (224), de préférence dans lequel l'élément de maintien de pompe d'alimentation comprend une entrée (280) dans le plateau inférieur (202) à une position au-dessous de la pompe.

13. Ensemble (1), comprenant
le support portable (200) selon l'une quelconque des revendications précédentes ; et
un contenant (300) contenant un aliment entéral ou un liquide.

14. Ensemble selon la revendication 13, comprenant en outre un ensemble d'alimentation (400) avec ou sans chambre d'égouttement (402) et/ou comprenant en outre une pompe d'alimentation (500).

15. Ensemble selon la revendication 13 ou 14, comprenant en outre une poche (100) qui contient les éléments énumérés dans l'une quelconque des revendications 13 ou 14, dans lequel la poche présente une base adaptée pour s'ajuster au plateau inférieur et/ou dans lequel la poche est dimensionnée pour recevoir étroitement la coque.
